# EUROPEAN PATENT APPLICATION

(11) **EP 1 332 767 A1**
(43) Date of publication of application: **06.08.2003**
(21) Application number: 03250412.8
(22) Date of filing: 22.01.2003
(51) Int. Cl.: A61M 5/315, A61M 5/30

(54) **Needleless injector with audible dosage mechanism**

(30) Priority: 25.01.2002 GB 0201717
(71) Applicant: The Medical House Plc, Attercliffe, Sheffield S9 2QJ (GB)
(72) Inventor: Stamp, Kevin, Sheffield, S35 1AR (GB)
(74) Representative: Stainthorpe, Vanessa Juliet

(57) **Abstract**

A needleless injector including an elongate main body, a dosage drum twistable about a longitudinal axis of said main body and an audible dosage mechanism, wherein the audible dosage mechanism is arranged to emit an audible indication of a dose being loaded into the injector.

## Description

This invention relates to the field of needleless injectors of the type used, for example, for subcutaneous injection of insulin, in particular a dosage mechanism therefor.

Needle-free injection devices or needleless injectors are designed to deliver a predetermined dose of a drug, for example insulin, by means of a spring-loaded or gas-powered syringe. One typical such needleless injector is described in US 5,782,802 [Vitajet Corp].

The device described in US 5,782,802 is designed to be easily refilled and reused by a single user with the correct dose of a medicament required at each injection. The device comprises basically of:
a) an injection head comprising a transparent thermoplastic generally cylindrical nozzle and a thermoplastic piston tip, both supplied to the user in a sterile pack;
b) a spring-powered unit with means to quickly attach and detach the injection head from this spring-powered unit; and
c) a pre-filled special vial (containing the medicament) which connects to the injection head, or alternatively an adaptor which is attached to a commercially-available vial of medicament.

A dose of medicament is loaded into the device of US 5,782,802 by turning a dosage drum in an anti-clockwise direction so as to draw a quantity of medicament into the nozzle. The user sets the dose by reading two dosage scales (at the same time) - amain scale on the body of the device which represents dose increments of 10 units and a circular scale on the dosage drum which represents increments of 1 unit. The readings from both scales need to be added to determine the total dose.

Since the scales are necessarily printed in relatively small type on the device, there is a risk that a visually-impaired user may set the dose incorrectly. Furthermore, a user having poor hand-eye coordination may also have difficulty in setting the dose correctly and, for all users, confusion may arise by the need to read two scales simultaneously.

There is thus a need for a needleless injector having a dosage mechanism which reduces the risk of the dose being incorrectly set. It is an object of the present invention to provide a needleless injector which alleviates the above-mentioned problems.

According to the present invention there is provided a needleless injector including an elongate main body, a dosage drum twistable about a longitudinal axis of said main body and an audible dosage mechanism, wherein the audible dosage mechanism is arranged to emit an audible indication of a dose being loaded into the injector.

Preferably, the dosage mechanism is arranged to emit audible clicks.

The audible clicks provide a visually-impaired user with a clear indication of the dose being loaded into the injector, thus reducing their reliance on reading a printed scale. Each click can be arranged to indicate a fixed quantity of the dose, for example one unit.

In a preferred embodiment, the audible dosage mechanism comprises a ratchet ring having a plurality of equispaced ratchet teeth and at least one tag which, in use, moves over said ratchet teeth so as to generate audible clicks. The spacing of the ratchet teeth can be set so as to indicate a fixed quantity of the dose. The volume of the click can be increased by providing a second tag (or even more than two tags), for example, with the second tag being placed adjacent the ratchet ring at 180 degrees from the first tag.

Preferably, said tag is fixed with respect to said dosage drum so that said twisting of the dosage drum causes the tag to move over the ratchet teeth.

In a preferred form, the audible indication is only generated when the dosage drum is twisted in an anti-clockwise direction. This reduces the likelihood of confusion by the user as to which way the dosage drum should be twisted in order to load the injector with a dose of a drug.

Preferably, said ratchet teeth and said tag are shaped so that, when the tag is moved in an anti-clockwise direction with respect to one of said ratchet teeth, the tag passes over said one of said ratchet teeth and drops onto an adjacent ratchet tooth, thus emitting an audible click.

Ideally, said ratchet teeth and said tag are shaped so that, when the tag is moved in a clockwise direction with respect to one of said ratchet teeth, the tag abuts a bluff end of an adjacent ratchet tooth, can move no further with respect to the ratchet teeth and hence "picks up" the ratchet ring which continues to move together with the tag in a clockwise direction.

Further preferably, said ratchet ring further comprises at least one internal ratchet tooth which, when the ratchet ring is moved in a clockwise direction, periodically passes over a detent which is fixed with respect to the main body of the injector so as to emit an audible click. In this way, an audible indication can be given to the user that the injector is being primed, i.e. when the dosage drum is twisted in a clockwise direction. The relatively slow frequency of clicks which are generated in a clockwise direction avoids confusion with the relatively fast frequency of clicks which are generated in an anti-clockwise direction when the injector is being loaded with a dose.

In a preferred embodiment, said dosage drum is provided with a window through which a single number representing the dose currently loaded into the injector can be viewed. Ideally, said window comprises a magnifying lens.

Preferably, said number is part of a spiral scale printed on said main body. In a preferred form the injector further comprises means for generating a verbal indication of said number.

Preferred embodiments of the present invention will now be more particularly described, by way of example only, with reference to the accompanying drawings wherein:
Figure 1 is a perspective view of a needleless injector;
Figure 2 is a side view of the injector of Figure 1;
Figure 3 is an exploded perspective view of the audible dosage mechanism;
Figure 4 is an assembled perspective view of the Figure 3 mechanism;
Figure 5 is a perspective view showing the interior of part of the dosage drum;
Figure 6 is a perspective view of the dosage drum, drawn to a larger scale; and
Figure 7 is a schematic perspective view showing the dosage values printed on the rearward end of the main body.

Throughout this description, the term "forward" refers to a direction toward the person who is injected by the device and "rearward" refers to a direction away from this person.

Although the preferred embodiment described below relates to a spring-powered needleless injector, other types of needleless injector may be encompassed by the invention. The invention is applicable in any injection device which requires the dose to be set by the user.

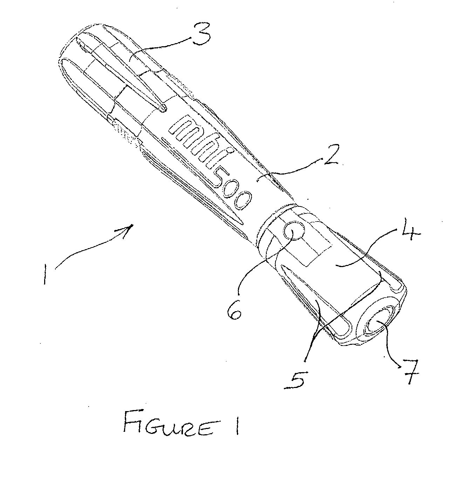

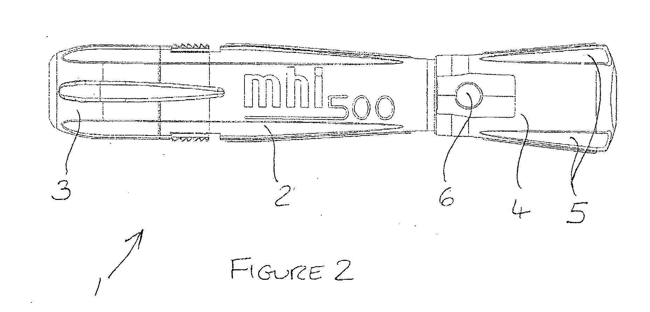

Referring generally to Figures 1 and 2, a needleless injector 1 comprises an elongate main body 2, at the forward end of which is a nozzle (not shown) covered, when the injector is not in use, by an end cap 3.

At the rearward end of the main body there is a dosage drum 4, having winding wings or other protrusions 5 which facilitate clockwise or anticlockwise twisting of the dosage drum 4 with respect to the main body 2 and which improve the user's grip thereon.

A window 6 is provided on the dosage drum 4, through which a user can read a scale indicating the dose of a drug loaded into the injector. This will be described in more detail below.

A discharge button 7 is situated on the rearward end of the dosage drum 4 and is used to effect delivery of the drug into the user.

In normal use, the injector 1 is held firmly in a vertical position and the dosage drum 4 is twisted as far as possible in a clockwise direction. A dose can then be loaded into the injector from a vial of the drug by twisting the dosage drum 4 in an anti-clockwise direction whilst observing the scale through the window 6.

Once the correct dose has been loaded, the dose is delivered into the user upon pressing the discharge button 7.

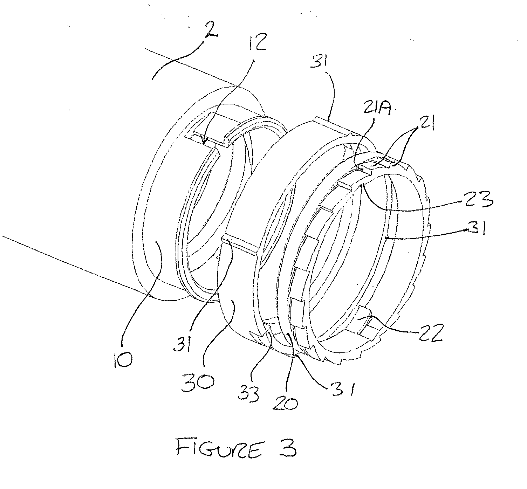

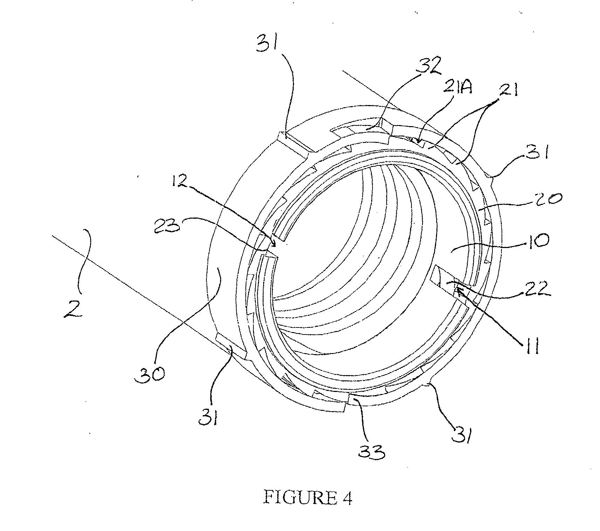

The needleless injector described herein is provided with an audible dosage mechanism which assists the user in loading the correct dose into the injector. The components of the audible dosage mechanism are shown in Figures 3 and 4.

Referring to Figure 3, the rearward end of the main body 2 is provided with an internally-threaded insert 10 which has two slots 11, 12 therein, located 180 degrees apart. The rearward part of the threaded insert is surrounded (when assembled) by a ratchet ring 20 which has external ratchet teeth 21 whose bluff ends 21A point in an anti-clockwise direction. When assembled, the slots 11, 12 lie adjacent the ratchet teeth 21. The ratchet ring 20 also has two internal ratchet teeth 22, 23 which are located 180 degrees apart. The ratchet ring 20 is surrounded (when assembled) by a ratchet pickup ring 30.

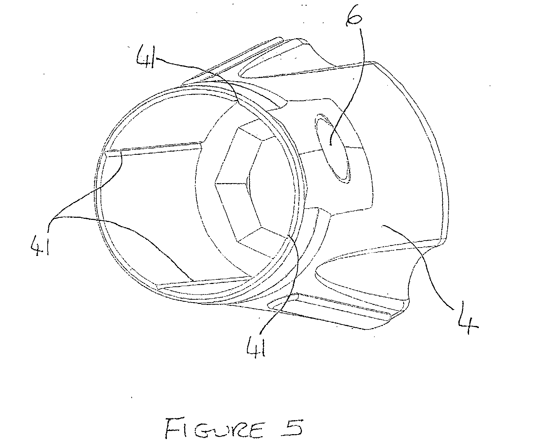

The pickup ring 30 is provided with four orthogonally-located splines 31 which, when the injector is fully assembled, locate into four orthogonally-located grooves 41 on the internal surface of the dosage drum 4 (illustrated in Figure 5). Thus the pickup ring 30 is rotationally fixed with respect to the dosage drum 4. The pickup ring is also provided with two depressed tags 32, 33 which are located at 180 degrees to one another and which can interact with (i.e. "pick up") ratchet teeth 21 on the ratchet ring 20. Alternatively, the tags 32, 33 may be integrally-formed with the dosage drum 4 as an alternative to using a pickup ring as described above.

The threaded insert 10 is a friction fit inside the rearward end of the main body 2 so that the insert 10 is rotationally fixed with respect to the main body 2. In an alternative embodiment (not illustrated), the threaded insert 10 may be replaced by an integrally-moulded part of the rearward end of the main body having an internal thread and two 180 degree spaced detents which serve the same function as slots 11, 12.

To summarise, the pickup ring 30 is rotationally fixed with respect to the dosage drum 4. The threaded insert 10 is rotationally fixed with respect to the main body 2. The ratchet ring 20 (which sits intermediate the two) is rotatable with respect to both in either direction.

When the dosage drum 4 is twisted in a clockwise direction, the pickup ring 30 must also rotate in a clockwise direction by virtue of the interlocking splines 31 and grooves 41. The tags 32, 33 each abut the bluff end 21A of a ratchet tooth, causing the ratchet ring 20 to rotate synchronously with the pickup ring 30 and dosage drum 4 in an anti-clockwise direction. Every 180 degrees, the internal ratchet teeth 22, 23 catch in the slots 11, 12, causing a clearly audible "click". This relatively slow clicking sound indicates to the user that the injector is being primed, ready for a dose to be loaded.

Once the injector is primed (i.e. by rotating the dosage drum 4 as far as possible in a clockwise direction), a dose can be loaded into the injector by rotating the dosage drum 4 in an anti-clockwise direction.

When the dosage drum is rotated in an anti-clockwise direction, the pickup ring 30 must also rotate in an anti-clockwise direction by virtue of the interlocking splines 31 and grooves 41. This causes the tags 32, 33 on the pickup ring 30 to disengage from the bluff end 21A of the adjacent ratchet tooth and to move over successive ratchet teeth 21 in an anti-clockwise direction whilst the ratchet ring 20 remains stationary with respect to the pickup ring 30. As the tags 32, 33 move over each successive ratchet tooth, a clearly audible "click" is heard. The spacing of the ratchet teeth 21 can be such that each click represents a specific volume of dose being drawn into the injector. For example, a click may be heard for every 18 degrees of anti-clockwise rotation, each click signifying that half a unit of insulin (or other drug) has been loaded.

The audible dosage mechanism means that a user can readily load the correct dosage, even if he/she has difficulty in reading the visual scale, simply by counting the relevant number of clicks. A much slower frequency of clicks when rotating the dosage drum in the opposite direction helps the user distinguish between priming (clockwise) and loading (anti-clockwise) the injector. It will be appreciated that the relative spacing of the external and internal ratchet teeth, the slots on the threaded insert and the depressed tags on the pickup ring can be varied in differently-sized devices, for example, where it may be required to calibrate the dosage scale differently.

So that no significant degrading of the clarity of the "clicks" occurs, the component parts need to have good resistance to wear. Acetyl may be used where good wear and self-lubricating properties are required.

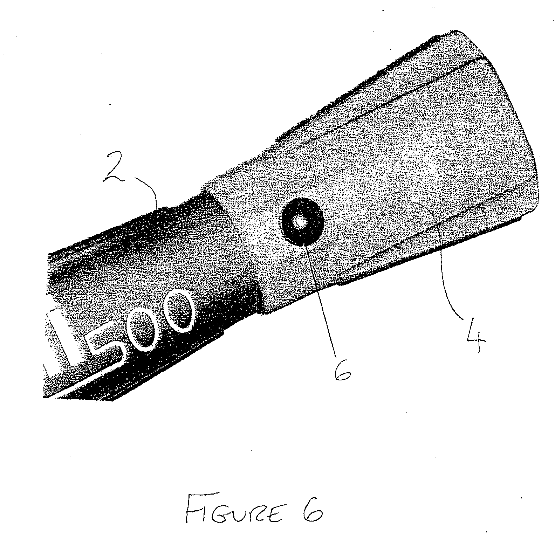

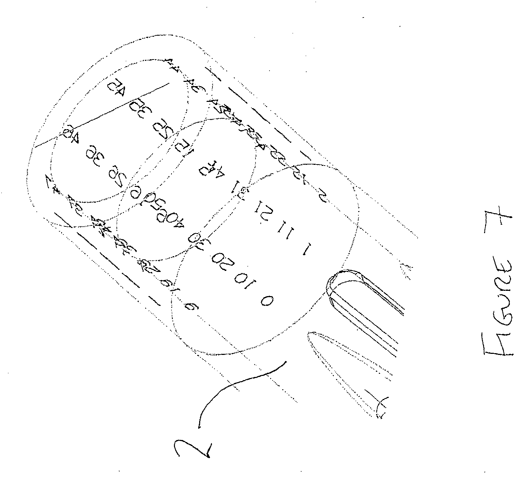

Referring now to Figures 6 and 7, the visual scale in the injector of the present invention has also been improved. The dosage drum 4 has a window 6 therein through which a scale indicating the dose can be read. The window is preferably a magnifying lens. The rearward part of the main body 2 which may be visible through the window 6 is printed with a number scale on a spiral so that only one number at a time is visible through the window, depending upon the relative position of the main body 2 and the dosage drum 4. For example, the numbers 0-50 may be printed on a right hand spiral with a pitch of 3.38mm, as illustrated in Figure 7.

As an alternative or additional feature, the dosage mechanism may be arranged to emit a verbal indication of the dose being loaded into the injector. For example, if the number scale shows "20" visible through window 6, the word "twenty" may be emitted by voice-synthesis electronics.

The injector of the present invention thus provides a much improved dosage mechanism whereby a visually-impaired user is given a clear indication of the size of dose being loaded into the injector. In this way, the danger of injecting an incorrect dose of a drug is reduced and the injector is more straightforward to use.

## Claims

1. A needleless injector including an elongate main body, a dosage drum twistable about a longitudinal axis of said main body and an audible dosage mechanism, wherein the audible dosage mechanism is arranged to emit an audible indication of a dose being loaded into the injector.

2. An injector as claimed in claim 1 wherein said dosage mechanism is arranged to emit audible clicks.

3. An injector as claimed in claim 1 or claim 2 wherein said audible dosage mechanism comprises a ratchet ring having a plurality of equispaced ratchet teeth and at least one tag which, in use, moves over said ratchet teeth so as to generate audible clicks.

4. An injector as claimed in claim 4 wherein said tag is fixed with respect to said dosage drum so that said twisting of the dosage drum causes the tag to move over the ratchet teeth.

5. An injector as claimed in any of the preceding claims wherein said audible indication is only generated when the dosage drum is twisted in an anti-clockwise direction.

6. An injector as claimed in claim 5 when dependent on claim 3 or claim 4 wherein said ratchet teeth and said tag are shaped so that, when the tag is moved in an anti-clockwise direction with respect to one of said ratchet teeth, the tag passes over said ratchet tooth and drops onto an adjacent ratchet tooth, thus emitting an audible click.

7. An injector as claimed in claim 6 wherein said ratchet teeth and said tag are shaped so that, when the tag is moved in a clockwise direction with respect to one of said ratchet teeth, the tag abuts a bluff end of an adjacent ratchet tooth, can move no further with respect to the ratchet teeth and hence "picks up" the ratchet ring which continues to move together with the tag in a clockwise direction.

8. An injector as claimed in claim 7 wherein said ratchet ring further comprises at least one internal ratchet tooth which, when the ratchet ring is moved in a clockwise direction, periodically passes over a detent which is fixed with respect to the main body of the injector so as to emit an audible click.

9. An injector as claimed in any of the preceding claims wherein said dosage drum is provided with a window through which a single number representing the dose currently loaded into the injector can be viewed.

10. An injector as claimed in claim 9 wherein said window comprises a magnifying lens.

11. An injector as claimed in claim 9 or claim 10 wherein said number is part of a spiral scale printed on said main body.

12. An injector as claimed in any of claims 9-11 further comprising means for generating a verbal indication of said number.
